# FASCICULE DE BREVET EUROPEEN

(11) **EP 1 016 652 B1**
(45) Date de publication et mention de la délivrance du brevet: **27.11.2002**
(21) Numéro de dépôt: 99403181.3
(22) Date de dépôt: 17.12.1999
(51) Int. Cl.: C07C 231/08

(54) **Procédé d'obtention de N-formyl-leucine de pureté élevée**
Verfahren zur Herstellung von hochreinem N-Formyl-Leucin
Process for the preparation of highly pure N-formyl-leucin

(30) Priorité: 30.12.1998 FR 9816611
(43) Date de publication de la demande: 05.07.2000
(73) Titulaire: ISOCHEM, 75194 Paris Cedex 04 (FR)
(72) Inventeur: Hussenet, Patricia, 91200 Athis Mons (FR); Le Goff, Philippe, 67000 Strasbourg (FR); Sennyey, Gérard, 91190 Saint-Aubin (FR); Vincent, Charles-Henry, 60460 Precy sur Oise (FR)
(74) Mandataire: Pech, Bernard

(56) Documents cités:
- EP-A- 0 080 119
- US-A- 4 789 757

## Description

L'invention concerne un procédé d'obtention de N-formyl-leucine de pureté élevée.

La N-formyl-leucine, constituée par la molécule de leucine dont un des atomes d'hydrogène fixé sur l'azote est remplacé par un groupe formyle, est un composé connu utilisé notamment pour réaliser des synthèses peptidiques.

Différents procédés de formylation d'amines, en particulier des groupes amines des amino-acides, sont connus. Industriellement, les amino-acides sont généralement N-formylés par action de l'acide formique ou d'un formiate de métal alcalin. Cependant ces procédés présentent des inconvénients. Des impuretés gênantes sont également formées, notamment du fait que la réaction est effectuée en présence d'anhydride acétique. Celui-ci réagit également sur les amino-acides et il se forme alors toujours une certaine quantité d'amino-acide N-acétylé qu'il est difficile de séparer de l'amino-acide N-formylé.

Un autre procédé de formylation a été décrit dans le brevet US n° 4 789 757. Il consiste à faire réagir l'amino-acide avec un très grand excès de formamide. Ainsi dans la majorité des exemples, une quantité molaire de formamide 10 fois supérieure à celle de l'amino-acide est utilisée. Les acides N-formyl-aspartique, N-formyl-glycine, N-formyl-alanine et N-formyl-phénylalanine sont ainsi préparés. Cependant, les méthodes pour isoler ces dérivés N-formylés du mélange réactionnel et pour les récupérer avec le moins possible d'impuretés ne sont pas décrites.

Pour un certain nombre d'applications telles que les applications dans le domaine pharmaceutique ou alimentaire, il est nécessaire de disposer d'une N-formyl-leucine de grande pureté. Si on réalise la formylation de la leucine par le procédé décrit précédemment au moyen de formamide, la N-formyl-leucine obtenue ne sera certes pas polluée par des impuretés telles que des dérivés N-acétylés, mais elle contiendra généralement toujours de la leucine n'ayant pas réagi, de l'isoleucine et d'autres impuretés présentes dans le produit de départ ainsi que les produits de réaction de ces impuretés avec le formamide.

L'objet de l'invention est par conséquent un procédé d'obtention de N-formyl-leucine ayant une pureté au moins égale à 98%.

Le procédé selon l'invention consiste à préparer la N-formyl-leucine par réaction de la leucine avec le formamide et est caractérisé en ce que, lorsque la réaction est terminée, on fait précipiter la N-formyl-leucine, à une température d'environ 0° à 40°C, en mélangeant le milieu réactionnel avec de l'eau et un acide de façon à ce que le pH final du mélange soit d'environ 2 à 3.

On a également trouvé que le procédé est amélioré si on effectue la réaction de la leucine avec le formamide en présence d'acide formique et notamment en présence d'environ 0,1 à 2 moles d'acide formique par mole de leucine.

Le procédé de l'invention permet d'obtenir la N-formyl-leucine avec la pureté souhaitée, au moins égale à 98%, et avec un bon rendement généralement de l'ordre de 80 à 90%.

La leucine que l'on utilise comme composé de départ est un composé connu qui se trouve dans le commerce sous les formes D, L et DL. Il contient généralement comme impureté prépondérante de l'isoleucine.

La réaction de N-formylation peut être réalisée comme indiqué dans le brevet US n° 4 789 757 avec un considérable excès de formamide, généralement avec environ 10 moles de formamide par mole d'amino-acide.

Cependant, on a maintenant trouvé qu'il est préférable de ne pas utiliser un très grand excès de formamide et qu'une quantité de formamide inférieure à 4 moles par mole de leucine et en particulier d'environ 1,5 à 3,5 moles par mole de leucine convient bien.

Il est aussi avantageux d'effectuer la réaction de N-formylation en présence d'acide formique. De préférence, on utilise environ 0,5 à 1 mole d'acide formique par mole de leucine.

La réaction est effectuée en chauffant le mélange constitué par le formamide et la leucine ainsi qu'éventuellement l'acide formique, à une température comprise entre environ 80°C et 110°C, de préférence entre environ 90°C et 100°C et de préférence sous atmosphère inerte telle que sous atmosphère d'azote. Elle dure en général de une à plusieurs heures.

Lorsqu'elle est terminée, si on utilise une quantité de formamide supérieure à la quantité préférée, il est alors avantageux de distiller une partie du formamide restant dans le milieu après la réaction, de façon à ne pas compliquer l'opération de précipitation en raison de cet excès de réactif.

On fait ensuite précipiter la N-formyl-leucine. Pour ce faire, on utilise une quantité importante d'eau, de préférence comprise entre environ 1,5 et 5 parties par partie en poids de formamide de départ, et un acide, en particulier un acide minéral, par exemple l'acide chlorhydrique ou sulfurique, de façon à ce que la fin de la précipitation s'effectue à un pH d'environ 2 à 3, de préférence d'environ 2 à 2,5.

Selon une variante, pour effectuer l'opération de précipitation, on ajoute tout d'abord de l'acide dans l'eau nécessaire à la précipitation pour que son pH soit acide, de préférence d'environ 2 à 3, puis on mélange le milieu réactionnel et l'eau acidifiée, de préférence en versant le milieu réactionnel dans l'eau acidifiée ou en versant simultanément le milieu réactionnel et l'eau acidifiée dans un réacteur. Pendant toute la durée de la précipitation, on maintient de préférence le mélange à un pH d'environ 2 à 3, et plus particulièrement d'environ 2 à 2,5 en ajoutant de l'acide dans le mélange si nécessaire. Selon cette variante, il est aussi avantageux, avant d'effectuer l'opération de précipitation, de diluer le mélange réactionnel avec de l'eau, par exemple en quantité comprise entre environ 0,2 et 0,4 partie par partie en poids de formamide.

Selon une autre variante, on mélange le milieu réactionnel tout d'abord avec l'eau puis on amène et on maintient le pH du mélange à environ 2 à 3, de préférence à environ 2 à 2,5, par ajout de l'acide.

L'acide utilisé est plus particulièrement l'acide sulfurique.

Il est également important que la température pendant la précipitation soit maintenue entre environ 0°C et 40°C, de préférence entre environ 5°C et 20°C.

La N-formyl-leucine cristallise spontanément mais on peut aussi introduire un germe pour favoriser la cristallisation.

La cristallisation terminée, on récupère les cristaux de N-formyl-leucine au moyen d'une des méthodes habituelles. Les cristaux obtenus ont notamment une taille suffisante pour être filtrés, lavés et séchés facilement.

Le rendement en N-formyl-leucine sèche est généralement supérieur à 80%.

La pureté de la N-formyl-leucine est égale ou supérieure à 98%, souvent même supérieure à 99%.

La N-formyl-leucine obtenue selon le procédé de l'invention peut être directement utilisée comme intermédiaire pour effectuer des synthèses de peptides ou pour préparer des produits pharmaceutiques.

Les exemples qui suivent illustrent l'invention sans toutefois la limiter.

### Exemple 1

Dans un réacteur muni d'un système d'agitation, on introduit, sous atmosphère d'azote, 165,2 g de L-leucine et 821,7 g de formamide. On chauffe le mélange à 100°C pendant environ 6 heures. Puis on élimine environ 513 g de formamide par distillation sous une pression de 20-30 mm de mercure et à une température de masse de 50°-60°C. On ajoute ensuite 1082 g d'eau dans le mélange. On le refroidit à 20°-25°C. Puis on l'acidifie à pH 2,5 avec de l'acide sulfurique. On l'agite 2 heures à environ 20°C puis une heure à 5°C. On filtre et on rince le gâteau avec 100 g d'eau à pH 2. On le sèche sous vide. On obtient ainsi 180,3 g de N-formyl-L-leucine (rendement 90%) de pouvoir rotatoire [α]_{D}²⁰ = -17,7° (C = 1 dans éthanol), soit une pureté de 98,3%.

Par chromatographie (CCM ou CPG), on détermine qu'il reste moins de 0,1% en poids de leucine et moins de 0,01% en poids d'isoleucine.

### Exemple 2

Dans un réacteur muni d'un système d'agitation, on introduit, sous atmosphère d'azote, 520 kg (11,55 kmol) de formamide et 500 kg (3,81 kmol) de L-leucine. On chauffe le mélange à une température comprise entre 96°C et 101°C et on le maintient à cette température pendant 10 heures. Il se transforme en une solution jaune-orange limpide. On ajoute alors 150 litres d'eau permutée, à environ 20°C. Puis on fait précipiter la N-formyl-L-leucine, en versant en environ 1 heure le mélange dans 1500 litres d'eau acidifiée jusqu'à un pH de 2 à 3 au moyen d'acide sulfurique à 55%, en maintenant ce pH pendant toute la durée de l'opération par ajout d'acide sulfurique à 55% et en maintenant la température entre 0°C et 35°C.

La N-formyl-L-leucine cristallise progressivement. Lorsque tout le mélange a été versé dans l'eau, on maintient encore 4 heures, le milieu à un pH de 2 à 2,5 et à une température de 15°C.

On recueille les cristaux formés au moyen d'une essoreuse, on les lave plusieurs fois avec de l'eau neutre et on les sèche en étuve. On obtient 490 kg (rendement 81%) de N-formyl-L-leucine ayant les caractéristiques suivantes :
Taux d'eau : 0,08%,
Point de fusion : 139°C,
Pureté : 99,8% (déterminée par chromatographie liquide haute pression, HPLC ou par titration).

### Exemple 3

Dans un réacteur muni d'un système d'agitation, on introduit, sous atmosphère d'azote, 1 kg de L-leucine, 0,68 kg de formamide et 0,35 kg d'acide formique.

On chauffe le mélange à environ 90°C pendant 3 à 4 heures. On ajoute ensuite 2 litres d'eau. On refroidit le mélange à 15°C.

Puis on introduit, dans le mélange, 0,62 kg d'acide sulfurique à 55% et on obtient un pH de 2,3. On continue à agiter le mélange, 30 minutes à 15°C. On recueille par essorage les cristaux formés, on les lave plusieurs fois avec de l'eau et on les sèche. On obtient 1,05 kg (rendement 86%) de N-formyl-L-leucine ayant une pureté de 98% (déterminée par HPLC).

## Revendications

1. Procédé d'obtention de N-formyl-leucine par réaction de la leucine avec le formamide, **caractérisé en ce que**, une fois la réaction terminée, on fait précipiter la N-formyl-leucine, à une température d'environ 0° à 40°C, en mélangeant le milieu réactionnel avec de l'eau et un acide de façon à ce que le pH final du mélange soit d'environ 2 à 3.

2. Procédé selon la revendication 1, **caractérisé en ce qu'**on effectue la réaction de la leucine avec le formamide en présence d'acide formique.

3. Procédé selon la revendication 1 ou 2, **caractérisé en ce que** la quantité de formamide mis à réagir avec la leucine est inférieure à 4 moles par mole de leucine.

4. Procédé selon l'une quelconque des revendications précédentes, **caractérisé en ce que** la quantité d'eau utilisée pour effectuer la précipitation est comprise entre environ 1,5 et 5 parties par partie en poids de formamide de départ.

5. Procédé selon l'une quelconque des revendications précédentes, **caractérisé en ce que**, pour effectuer la précipitation, on acidifie l'eau nécessaire à la précipitation par ajout d'acide, avant de mélanger l'eau au milieu réactionnel.

6. Procédé selon la revendication 5, **caractérisé en ce que**, avant d'effectuer l'opération de précipitation, on dilue le mélange réactionnel avec de l'eau.

7. Procédé selon l'une quelconque des revendications 1 à 4, **caractérisé en ce que**, pour effectuer la précipitation, on mélange d'abord l'eau avec le milieu réactionnel puis on amène et on maintient le pH du mélange à environ 2 à 3, par ajout d'acide.

8. Procédé selon l'une quelconque des revendications précédentes, **caractérisé en ce que** pendant l'opération de précipitation, le pH est au moins à la fin maintenu entre environ 2 et 2,5.

9. Procédé selon l'une quelconque des revendications précédentes, **caractérisé en ce que** la température de l'étape de précipitation est comprise entre environ 5°C et 20°C

10. Procédé selon l'une quelconque des revendications précédentes, **caractérisé en ce que** la leucine et le formamide sont mis à réagir à une température comprise entre environ 80°C et 110°C.

11. Procédé selon l'une quelconque des revendications précédentes, **caractérisé en ce que** la réaction est effectuée en présence d'environ 0,1 à 2 moles d'acide formique par mole de leucine.

## Patentansprüche

1. Verfahren zur Herstellung von N-Formyl-leucin durch Umsetzung von Leucin mit Formamid, **dadurch gekennzeichnet, daß** nach abgeschlossener Reaktion das N-Formyl-leucin bei einer Temperatur von etwa 0 bis 40 °C gefällt wird, indem das Reaktionsmedium mit Wasser und einer Säure so vermischt wird, daß der pH-Wert des Gemisches bei etwa 2 bis 3 liegt.

2. Verfahren nach Anspruch 1, **dadurch gekennzeichnet, daß** die Umsetzung von Leucin und Formamid in Gegenwart von Ameisensäure durchgeführt wird.

3. Verfahren nach Anspruch 1 oder 2, **dadurch gekennzeichnet, daß** die mit dem Leucin umgesetzte Formamidmenge unter 4 mol pro Mol Leucin liegt.

4. Verfahren nach einem der vorhergehenden Ansprüche, **dadurch gekennzeichnet, daß** die zum Ausfällen verwendete Wassermenge im Bereich von etwa 1,5 bis 5 Teile auf 1 Gewichtsteil anfänglich eingesetztes Formamid liegt.

5. Verfahren nach einem der vorhergehenden Ansprüche, **dadurch gekennzeichnet, daß** bei der Durchführung der Fällung das zum Ausfällen erforderliche Wasser durch Zugabe einer Säure angesäuert wird, bevor das Wasser mit dem Reaktionsmedium vermischt wird.

6. Verfahren nach Anspruch 5, **dadurch gekennzeichnet, daß** das Reaktionsmedium vor der Fällung mit Wasser verdünnt wird.

7. Verfahren nach einem der Ansprüche 1 bis 4, **dadurch gekennzeichnet, daß** zur Durchführung der Fällung zunächst das Wasser mit dem Reaktionsmedium vermischt wird und anschließend der pH-Wert des Gemisches durch Zugabe einer Säure auf etwa 2 bis 3 eingestellt und dort gehalten wird,

8. Verfahren nach einem der vorhergehenden Ansprüche, **dadurch gekennzeichnet, daß** während des Arbeitsgangs des Fällens der pH-Wert zumindest am Schluß im Bereich von etwa 2 bis 2,5 gehalten wird.

9. Verfahren nach einem der vorhergehenden Ansprüche, **dadurch gekennzeichnet, daß** die Temperatur bei der Fällung im Bereich von etwa 5 bis 20 °C liegt.

10. Verfahren nach einem der vorhergehenden Ansprüche, **dadurch gekennzeichnet, daß** das Leucin und das Formamid bei einer Temperatur im Bereich von etwa 80 bis 110 °C umgesetzt werden.

11. Verfahren nach einem der vorhergehenden Ansprüche, **dadurch gekennzeichnet, daß** die Umsetzung in Gegenwart von etwa 0,1 bis 2 mol Ameisensäure pro Mol Leucin erfolgt.

## Claims

1. Process for obtaining N-formylleucine by reacting leucine with formamide, **characterized in that**, once the reaction is complete, the N-formylleucine is made to precipitate out, at a temperature from about 0° to 40°C, by mixing the reaction medium with water and an acid such that the final pH of the medium is about 2 to 3.

2. Process according to Claim 1, **characterized in that** the reaction of leucine with formamide is carried out in the presence of formic acid.

3. Process according to Claim 1 or 2, **characterized in that** the amount of formamide reacted with the leucine is less than 4 mol per mole of leucine.

4. Process according to any one of the preceding claims, **characterized in that** the amount of water used to effect the precipitation is between about 1.5 and 5 parts per part by weight of starting formamide.

5. Process according to any one of the preceding claims, **characterized in that**, in order to effect the precipitation, the water required for the precipitation is acidified by adding acid, before mixing the water with the reaction medium.

6. Process according to Claim 5, **characterized in that**, before carrying out the precipitation operation, the reaction mixture is diluted with water.

7. Process according to any one of Claims 1 to 4, **characterized in that**, to effect the precipitation, the water is first mixed with the reaction medium and the pH of the mixture is then brought to and maintained at about 2 to 3, by adding acid.

8. Process according to any one of the preceding claims, **characterized in that**, during the precipitation operation, the pH is, at least at the end, maintained between about 2 and 2.5.

9. Process according to any one of the preceding claims, **characterized in that** the temperature of the precipitation step is between about 5°C and 20°C.

10. Process according to any one of the preceding claims, **characterized in that** the leucine and the formamide are reacted together at a temperature of between about 80°C and 110°C.

11. Process according to any one of the preceding claims, **characterized in that** the reaction is carried out in the presence of about 0.1 to 2 mol of formic acid per mole of leucine.
